# EUROPEAN PATENT SPECIFICATION

(11) **EP 1 173 464 B1**
(45) Date of publication and mention of the grant of the patent: **18.06.2008**
(21) Application number: 00922365.2
(22) Date of filing: 27.04.2000
(51) Int. Cl.: C07J 9/00, A61K 31/575, C11B 13/00, C11B 13/02, A61P 3/06

(54) **Process for preparing a phytosterol composition**
Verfahren zur Herstellung einer Phytosterolzusammensetzung
Procédé pour la préparation d'une composition phytosterol

(30) Priority: 27.04.1999 US 300135
(43) Date of publication of application: 23.01.2002
(73) Proprietor: Forbes Medi-Tech Inc., Vancouver, British Columbia V6C 2T8 (CA)
(72) Inventor: COSS, James, L., Lion's Bay, British Columbia V0N 2E0 (CA); KUTNEY, James, P., Vancouver, British Columbia V6L 3C7 (CA); MILANOVA, Radka, K., Vancouver, British Columbia V6G 1E1 (CA); JOLLEZ, Paul, Sherbrooke, Quebec J1J 1G9 (CA)
(74) Representative: Jones, Helen M.M.
(86) International application number: PCT/CA2000/000455
(87) International publication number: WO 2000/064921

(56) References cited:
- EP-A- 1 177 729
- WO-A-00/61771
- WO-A-01/53320
- WO-A-98/01759
- WO-A-99/32097
- HALLIKAINEN MAARIT A ET AL: "Effects of 2 low-fat stanol ester-containing margarines on serum cholesterol concentrations as part of a low-fat diet in hypercholesterolemic subjects" AMERICAN JOURNAL OF CLINICAL NUTRITION, vol. 69, no. 3, March 1999 (1999-03), pages 403-410, XP002279703 ISSN: 0002-9165

## Description

This invention relates to the purification of phytosterols from wood or plant derived sources.

### BACKGROUND OF THE INVENTION

Sterols are naturally occurring compounds that perform many critical cellular functions. Phytosterols such as campesterol, stigmasterol, campestanol and beta-sitosterol in plants, ergosterol in fungi and cholesterol in animals are each primary components of cellular and sub-cellular membranes in their respective cell types. The dietary source of phytosterols in humans comes form plant material i.e. vegetables and plant oils. The average Western diet contains about 60-80 mg of phytosterols per day, which can be contrasted with a vegetarian diet, which provides about 500 mg per day. Recently, these dietary plant sterols have received a great deal of attention because of their possible anti-cancer properties and their ability to decrease cholesterol levels when fed to a number of mammalian species, including humans.

Chemically, phytosterols closely resemble cholesterol in structure. The major phytosterols are beta-sitosterol, campesterol and stigmasterol. Others include stigmastanol (beta-sitostanol), sitostanol, desmosterol, chalinasteral, poriferasterol, clionasterol and brassicasterol. The chemical structures of beta-sitosterol, campesterol, campestanol, brassicasterol, brassicastanol and sitostanol are as follows:

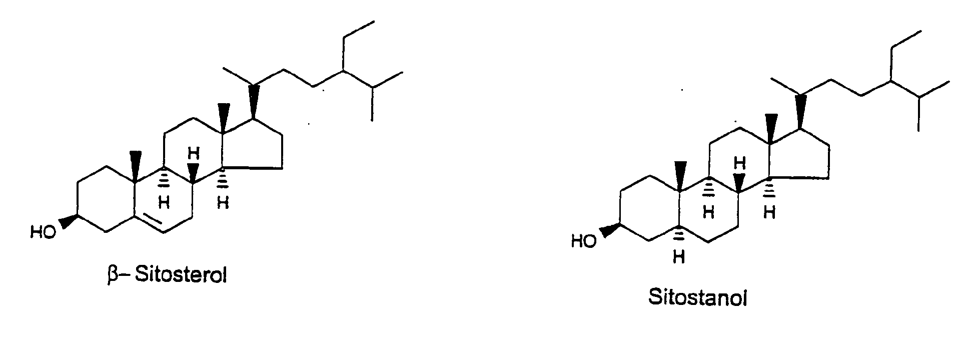

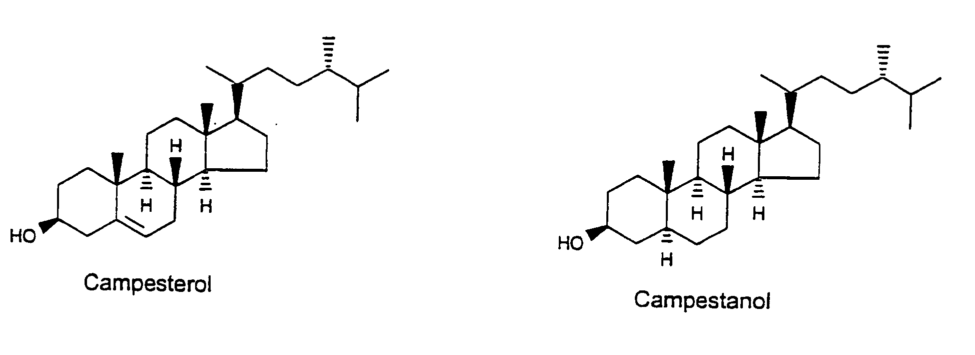

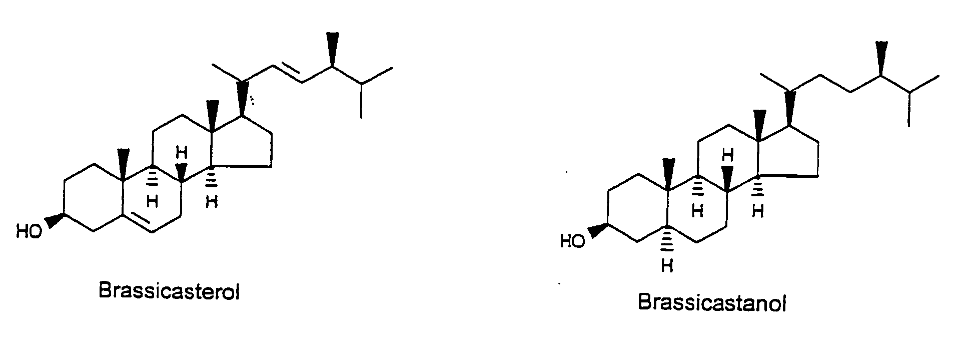

The mechanism by which phytosterols lower blood cholesterol in animals is unclear, but it appears to involve the inhibition of cholesterol absorbtion from the proximum jejunum by competing with cholesterol at specific uptake sites. Research data has also suggested that some phytosterols are not absorbed at the proximal jejunum at all (sitostanol) and, when there is absorbtion (beta-sitosterol), it is in very limited quantities.

Based on these research findings, the use of phytosterols as a dietary supplement to reduce cholesterol absorbtion has been investigated. Lees et al., *supra;* Pollak, Pharmac. Ther., 31 (1985) 177-208; Raicht et al., Biochimica et Biophysica Acta, 388 (1975) 374-384.

In Lees et al., *supra,* a comparison was made between the effects of siterosterol preparations from two sources, soy sterols and tall oil sterols, on plasma cholesterol. Plant sterol preparations were found to be effective in treating patients with hypercholesterolemia. Pollak, *supra,* is a survey paper of phytosterols and their effect on serum lipids. Raicht, *supra,* describes further the effect of beta-sitosterol on sterol balance and rate-limiting enzymes of sterol metabolism. More recently, in US Patent No. 5,770,749 to Kutney et al., a unique blend of phytosterols comprising beta-sitosterol, campesterol and stigmastanol was found to be particularly effective in lowering serum cholesterol.

It is generally accepted that phytosterols offer a unique combination of long-term safety, efficacy, and versatility in human treatment. The ongoing challenges with respect to phytosterols is in their isolation and purification from plant sources, in determining additional sources which are cost-effective and manageable on a large-scale.

Traditionally, phytosterols have been isolated from sources such as corn oil, wheat germ oil, soya bean pitch and corn oil pitch. Similarly, tall oil pitch, which is obtained during the process of preparing paper from wood, particularly pine wood, has been used as a phytosterol source. Generally, in this process, wood chips are digested with caustic soda to produce a pulp and "soap". After acidulation of the soap, the resultant tall oil is then distilled to remove the volatile materials leaving a "pitch" as the residue. It is from this pitch that researchers have traditionally isolated phytosterols.

United States Patent No. 3,840,570 to Julian provides a process for preparing sterols from tall oil pitch by extraction in a water - alcohol - hydrocarbon mixture followed by saponification and subsequent purification. The starting material in this process is tall oil pitch from which are extracted phytosterols and various impurities. It is recognized that, in any tall oil pitch purification process, the long-chain alcohol and acid impurities are particularly difficult to separate from the sterols (which are, themselves, high molecular weight alcohols).

Other researchers have addressed the issue of sterol purification from tall oil pitch: United States Patent No. 2,835,682 to Steiner and Fritz; United States Patent No. 2,715,638 to Albrecht and Herrlinger; United States Patent No. 2,573,891 to Christenson. It is important to note that in each of these known purification processes, the starting material was tall oil pitch, which has the recovery problems discussed above.

US Patent No. 4,044031 to Johansson et al. describes a hexane extraction process for the removal of sterols from crude sulfate soap skimmings. US Patent No. 3,965,085 to Kaukas uses, for the isloation of phytosterols from soap, a mixture of solvents comprising hexane, acetone, methanol and water. US Patent No. 5,770,749 to Kutney et al. teaches a process of extracting sterols from pulping soap in which the solvent mixture comprises water, ketone, a hydrocarbon and no alcohol.

The aforementioned patents are either limited to extraction from one of soap or pitch (but not both) and focus on the "extraction" phase while subsequent purification is achieved simply by known techniques such as crystallization.

British Patent No. 1,164,769 describes a method for isolating sterols from mixtures with lipids wherein the mixture is dissolved, preferably in a hydrocarbon solvent, the solution is mixed with an aqueous solution of a metal salt which is suitable for complex formation, the water is progressively removed by azeotropic distillation, and the precipitated adduct is isolated and split in the conventional manner after cooling of the mixture.

This method, and ones like it (German Patent No. 827,199) have the disadvantage that they are technically very costly due to the high reaction temperature required (usually over 100°C) and that in the isolation of many 3-hydroxy steroids and 3-oxo steroids, considerable loss of product is experienced, since these steroids are destroyed under these conditions. Furthermore, these methods have the disadvantage that the recovery of the metal salt used for the adduct formation (recovery being necessary in large scale production given environmental considerations) is often very costly.

W09801759 discloses phytosterol compositions and their effect as cholesterol lowering agents. The ratio of cam pesterol/β-sitosterol is optimised and the level of hydrogenated compounds, campestanol and stigmastanol is also mentioned.

Hallikainen, M., et al in Am. J. Clin. Nutrition, 69(3), 403-4101999, describe the effects of plant sterols on serum total and LDL-cholesterol levels.

It is an object of the present invention to obviate or mitigate these and other disadvantages by providing a unique purification process which can be used in the isolation of phytosterols from either soap or pitch and which likewise can be used in the isolation of phytosterols from other plant sources.

### SUMMARY OF THE INVENTION

The present specification discloses a universal process for purifying phytosterols from a wood or plant derived source which comprises extracting from the source a concentrated extracted comprising phytosterols and a hydrocarbon; complexing the extract so formed with a metal salt; separating the phytosterol/metal salt complex from the hydrocarbon; washing the complex with a solvent mixture comprising one or both of a hydrocarbon and a ketone; hydrolysing the washed complex so formed and finally separating the phytosterols therefrom.

The present specification discloses compositions of phytosterols comprising beta-sitosterol, campesterol, campestanol and sitostanol which are useful in preventing or treating primary and secondary dyslipidemias and atherosclerosis including coronary heart disease, peripheral vascular disease and strooke in humans and other animals.

The present specification discloses a method of treating or preventing primary and secondary dyslipidemias and atherosclerosis including coronary heart disease, peripheral vascular disease and stroke in humans and other animals which comprises administering to the animal the composition of the present invention.

The present invention provides a process of preparing a phytosterol composition which comprises blending a mixture of both hydrogenated and non-hydrogenated phytosterols, from various wood and plant derived sources. The process is for preparing a phytosterol composition having from 30-80% beta-sitosterol, 4-25% campesterol, 10-60% sitostanol and 5-30% campestanol and campestanol which comprises:
a) hydrogenating phytosterols from a first source to form an intermediary product comprising from 60-85% beta-sitostanol and from 20-40% campestanol; and
b) blending this intermediary product with phytosterols derived from a second source:

Although it is known to produce some types of phytosterols from the either the pitch or from the soap per se, there has not heretobefore been disclosed an effective process of purifying phytosterols from both. This has been the case because pitch, as described below, is significantly different in composition from soap. The complexation-hydrolysis purification process is suitable for phytosterol preparation from either. In addition, and quite surprisingly, it has been found that this purification process is equally suited to the purification of phytosterols from plant and particularly vegetable sources. Accordingly, a "universally" applicable purification process is described herein.

What has also been found, in accordance with the present invention, are unique compositions that exhibit excellent results in lowering total serum cholesterol (TC) and low density lipoprotein (LDL) serum cholesterol while at the same time, maintaining or elevating the serum levels of high density lipoprotein (HDL) cholesterol.

### BRIEF REFERENCE TO THE DRAWINGS:

Various aspects of the invention will be illustrated by the following non-limiting drawings wherein:
Figure 1 is a flow chart of a purification process from either soap, pitch or plant extract in accordance with the present invention; and
Figure 2 is a schematic diagram showing the refining of vegetable oil.

### PREFERRED EMBODIMENTS OF THE INVENTION

The present disclosure provides a unique complexation-hydrolysis process for purifying phytosterols from many varied sources, including wood derived sources (both soap and pitch) and from plant sources. The process comprises extracting from the source a concentrated extract comprising phytosterols and a hydrocarbon; complexing the extract so formed with a metal salt; separating the phytosterol/metal salt complex from the hydrocarbon; washing the complex with a solvent mixture comprising one or both of a hydrocarbon and a ketone; hydrolysing the washed complex so formed and finally separating the phytosterols therefrom.

### Soap vs. Pitch

During the processing of wood chips with an alkaline solution like sodium hydroxide, the neutral components float to the top of the black liquor and form a layer generally referred to as the "soap". Upon acidulation of the soap, followed by distillation of the volatile constituents, a dark, tarry residue remains. This residue is referred to as the "pitch".

From a technical perspective, of all the wood or forestry derived products, it is the soap and the pitch that are most suitable for the isolation and purification of phytosterols. Both the soap and the pitch have their advantages and disadvantages as starting materials. During high temperature distillation of tall oil, as discussed above, some thermal decomposition of the volatile constituents or phytosterols may occur, with the result that these decomposed substances remain in the pitch. Nonetheless, two advantages of the pitch which cannot be overlooked are that it has a higher sterol concentration than the soap (up to 11% of its mass) and it has an up to six times smaller volume in comparison with the soap which makes it more economical to work with. Within the scope of the present invention, there is provided a purification process which is effective to produce good yields of phytosterols from the pitch at the high purities required for pharmaceutical and food uses.

Conversely, soap contains about 1-4% of unbound free phytosterols and few of the decomposition products found in the pitch. Generally, the soap is prepared by a known process (the "Kraft" process), wherein wood chips are treated with caustic soda. The wood chips for use in preparing either the pitch or soap for use in accordance with the present invention may be derived from any hard wood or soft wood variety of tree including, but not limited to, fir, cedar, pine, spruce, oak, hemlock and poplar. Most preferably, the chips are derived from any Pacific Northwest American or European forest variety of woods

### Soap and Pitch Extraction Phase

To some extent, the extraction phase will differ depending on whether the starting material is the soap or the pitch and it is well within the skill of those in this field to choose an appropriate extraction procedure. Either way, it is contemplated within the present invention that the "product" of the extraction phase will be a concentrated extract comprising phytosterols, for example a hexane layer achieved through solvent extraction. This product of the extraction phase then undergoes the novel purification process of the present invention.

While these are not being claimed as part of the present invention, there are a number of extraction procedures known in the art which are entirely suitable for use herein. For example, with respect to soaps, the phytosterols may be extracted (and concentrated extract produced) as described in US Patent No. 5,770,749 to Kutney et al, the contents of which are incorporated herein by reference. Therein, a soap is mixed with a ketone and water solution. A hydrocarbon solvent is used to extract the sterols. This step can be performed at temperatures generally from about 25°C to about 150°C, but most preferably from about 50°C to about 100°C. Most preferably, this extraction phase is continued over 15 to 24 hours. It is important to note that the use of alcohol is neither required nor suggested during the extraction phase. The extraction process is conducted using a ketone-water-hydrocarbon solvent wherein the ketone is most preferably acetone and the hydrocarbon is most preferably hexane.

The product of the extraction phase is a concentrated extract or residue that is then purified in accordance with the process of the present invention.

### Plant Source Extraction Phase

There are many excellent plant sources of phytosterols and phytostanols. For example, phytosterols are present in vegetables, vegetable oils (such as corn oil, and canola or rapeseed oil), wheat extracts such as wheat germ oil, soy extracts, rice extracts, rice bran and sesame oil to name a few. Aquatic plants may also be processed to extract phytosterols. In accordance with the present invention, crude phytosterols may be extracted from any plant source and then subsequently purified using the novel process described and claimed herein.

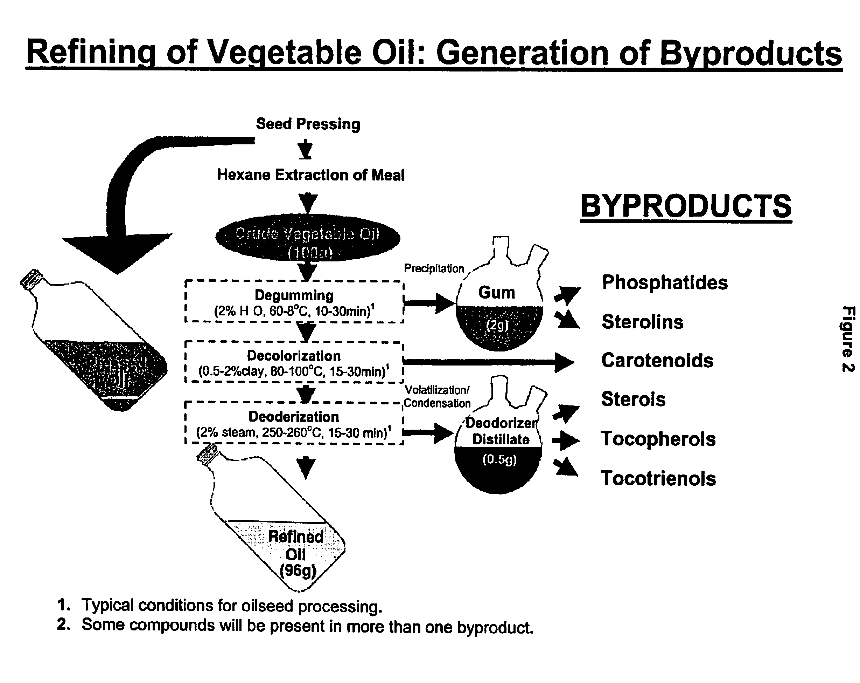

In a preferred form, the plant source is a vegetable extract, such as a vegetable oil distillate. Figure 2 represents a schematic outline of a process in which vegetable oil is refined thereby creating a by-product of a vegetable oil distillate. This distillate is suitable for purification using the process of the present invention.

The vegetable oil refining or "extraction" process operates basically as follows: the seeds are pressed to yield both crude pressed oil and meal. The latter is mixed with a hexane solvent to produce crude vegetable oil, which then undergoes a series of de-gumming, de-colourising and deodorising steps to yield, ultimately, the refined oil. A by product of the deodorization process is the vegetable oil or "deodorizer" distillate which is then purified using the complexation-hydrolysis procedure of the present invention.

### Purification Phase

The complexation-hydrotysis purification process generally comprises the following steps:
1.taking the product of the extraction phase, for example, the concentrated extract comprising phytosterols and a hydrocarbon and adding thereto a metal salt in order to form a complex between the metal salt and the phytosterols;
2. separating the complex so formed from the hydrocarbon;
3. washing the complex with a solvent mixture comprising one or both of a hydrocarbon and a ketone;
4. hydrolysing the washed complex; and
5. separating the phytosterols from the metal salt.

The metal salt may be selected from any halides of calcium, including, but not limited to anhydrous calcium chloride (CaCl2) or hydrated derivatives thereof, anhydrous calcium bromide (CaBr2) or hydrated derivatives thereof, and anhydrous calcium iodide (Cal2) or hydrated derivatives thereof. Other metal salts such as, for example, the halides of magnesium, manganese, zinc, copper, nickel, iron, radium, barium, strontium, beryllium may also be used. In a most preferred form, the molar equivalents of the metal salt as compared to molar equivalents of the phytosterols in the concentrated extract can vary from 1 to 10. In a preferred form of this process, CaCl2 is used as the metal salt due to its ready availability, and disposability i.e. a complex recycling process can be avoided due to its relatively low cost. In a most preferred form, anhydrous CaCl2 is used as the metal salt.

Preferably, the hydrocarbon in the solvent mixture is selected from the group comprising all C5 to C10 hydrocarbons or an ether such as ethyl ether or higher analogue thereof. Most preferably, the hydrocarbon is one or more of the following: hexane, petroleum ether, methyl isobutyl ketone (MIBK), acetone, or methyl ethyl ketone (MEK) .

Preferably, the ketone in the solvent mixture may have from C1 to C10 alkly groups, including all derivatives thereof. Preferably, the ketone is 2-propanone (acetone). Generally, the solvent comprises a mix of hexane and acetone, preferably in a ratio range of 1 to 20. Preferably, the ratios of extract to solvent mixture may vary from about 0.5 litres per kilogram of extract to 5 litres per kilogram of extract. Most preferably, about 3 litres of solvent mixture per kilogram of extract is used.

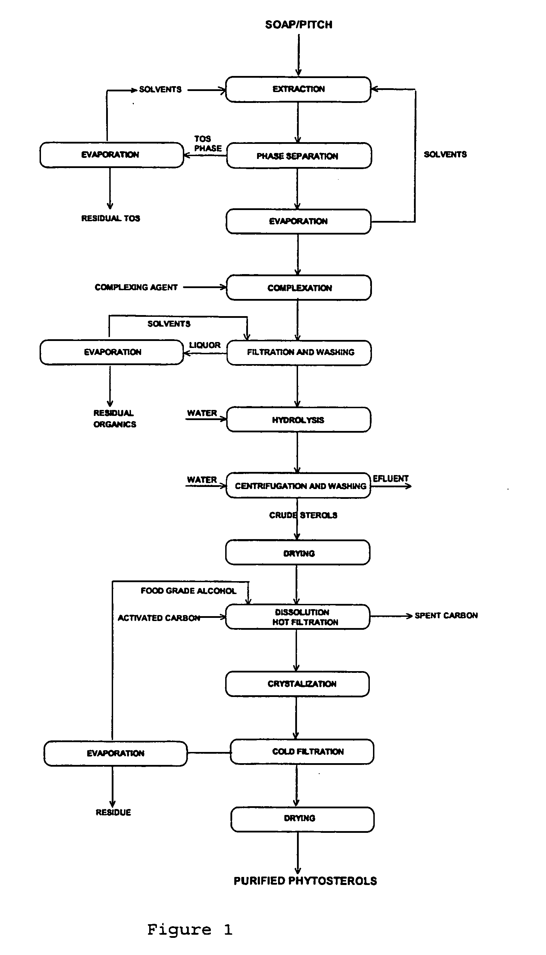

In a preferred form, the purification process (shown in Figure 1) works as follows: the metal salt is dissolved in water or alcohol (preferably methanol) and added to the concentrated extract (a hydrocarbon layer, which is most often a hexane layer depending on the prior extraction steps). The mixture is then agitated and heated to evaporate the water. After final extraction of the water from the mixture, the complexation reaction is complete and the complex is separated from the hydrocarbon layer, most preferably by centrifugation. Thereafter, the complex is washed in the centrifuge with fresh solvent comprising one or more of a hydrocarbon and ketone. In a centrifuge, the volume of washing can be very low, in the range of one to five times the volume of the residual hydrocarbon layer that wets the cake before washing.

The washed complex is then hydrolysed, most preferably by reacting it with hot water (in temperatures ranging from 50-100 degrees C). The residual solvent trapped in the complex is stripped and condensed. After final recovery of the residual solvent, the hydrolysis reaction is completed. During this reaction, the water dissolves the metal salt, concomitantly liberating the crude phytosterols. The crude sterols are then separated from the salty water, preferably through centrifugation. The volume of water may be very small, in the range of one to five times the volume of the residual salty water that wets the cake before washing. The phytosterols are then dried, preferably under vacuum, to a residual water content of 5% or less.

The recovery of phytosterols from the concentrated extract using this novel complexation-hydrolysis is in the range of 60 to 80% on a pure sterol basis. The major sterol content is in the range of between 60 to 80%.

Optionally, the phytosterols purified as above, may be subjected to a final purification phase. The dried sterol is dissolved in an alcohol, at boiling point and under reflux conditions. Generally, the amount of alcohol is from 5 to 10 litres for every kilogram of dried phytosterols, most preferably 9 litres. Suitable alcohols include all C1 to C6 alcohols and derivatives thereof. Most preferred is 2-propanol (isopropyl alcohol). A suitable adsorbent such as activated carbon or bleaching clay is then added to the solution. The amount of adsorbent can vary from 5 to 20 kilograms per kilogram of dried phytosterol.

The mixture so prepared is agitated under reflux for approximately one hour and hot filtered to recover the adsorbent. The alcohol is cooled down from about 78 degrees C to from 5 to 20 degrees C in order to crystallize the sterols which are then separated, for example in a centrifuge, and washed with fresh alcohol. The very pure phytosterols so formed are then dried under vacuum. The recovery from this phase is in the range of 70 to 80% on a pure sterol basis. This final product has a major sterol content higher than 86% and a total sterol content higher than 95%.

### Phytosterol Compositions

The phytosterol compositions disclosed herein comprise beta-sitosterol, campesterol, campestanol, and sitostanol and all natural or synthesised forms thereof and derivatives thereof, including isomers. The compositions per se, as well as the preferred concentration ratios of the constituent phytosterols, described in more detail below, are distinct from the sitostanol-based compositions which have been investigated by researchers to date. Generally, in a most preferred form of the present composition, both the sitostanol and campestanol concentrations are higher than ever taught or used by prior researchers. In addition, the beta-sitosterol concentration is significantly lower than that advocated in prior teachings. The end result, i.e. the combination of these four primary phytosterols in concert and most preferably, at the concentration ranges disclosed, is a composition which exhibits a marked ability to lower total serum and LDL cholesterol levels while concomitantly increasing both the beneficial HDL serum cholesterol levels and HDULDL ratio. It should be noted that, throughout this disclosure, the term "phytosterol" in many instances is intended to encompass both phytosterols and their saturated or hydrogenated counterparts "phytostanols". Limitations should not be read into the disclosure by the absence of the each repeated term.

In a preferred form, the composition formed by the purification process described herein comprises from 10 to 60% sitostanol, 4 to 25% campesterol, 5 to 30% campestanol, and 30 to 80% beta-sitosterol. Most preferably, the composition comprises from 10-30% campestanol. It is to be understood, however, that if the composition is prepared by the purification process, and depending on the original "source" from which the phytosterols are purified (i.e. soap, pitch or plant), additional phytosterols, such as brassicasterol and brassicastanol, may form part of the resultant "end product" and may contribute to the therapeutic efficacy.

For example, in extracting and purifying phytosterols and phytostanols from some vegetable sources such as rapeseed oil, significant concentrations of brassicasterol and brassicastanol are present in the resultant purified composition. Accordingly, in one preferred form of the composition of the present invention, along with the four phytosterols described above, there is included from 3-40% brassicasterol and/or from 3-40% brassicastanol, and more preferably from 3-20% of each.

The compositions may be produced by either one of the following means:
1. each of the constituent phytosterols may be synthesized *de novo* or extracted and purified from various sources (for example from vegetable sources) and then combined ; or
2. the composition may be prepared from wood or plant sources by using known extraction techniques followed by the novel purification process described herein.

In order to achieve compositions having the preferred phytosterol ratios as described above, a most preferred process is to combine steps 1 and 2 above, that is, to "blend" phytosterols and phytostanols from varying sources. This way, one may take advantage of particularly high levels of one sterol component from one source (for example sitosterol) while another source may be richer in another required component (for example sitostanol).

As described above, phytosterols and their hydrogenated or saturated counterparts called "phytostanols" are present in many natural sources, including forestry by-products and wood and plant sources. They may be procured from vegetable sources, from the processing of plant oils such as corn oil as shown in Figure 2 (and including aquatic plants), from wheat germ oil, soy extract, rice extract, rice bran, rapeseed oil, sesame oil and from fish oils.

In one preferred form, the blending process of the present invention comprises:
1) hydrogenating "plant" or "vegetable" derived phytosterols to yield a product comprising from 60-85% beta-sitostanol and from 20-40% campestanol; and
2) blending this intermediary product with the forestry-or wood derived phytosterol composition prepared in accordance with the purification process described herein to yield a most desired composition having the ratios as defined herein for optimal therapeutic efficacy.

Generally, the ratios of hydrogenated "vegetable or plant derived" phytosterols to wood-derived phytosterols can range from about 1 to 3. More preferably, hydrogenated plant derived phytosterols are blended in about a 1:2 ratio with the wood-derived phytosterol composition of the present invention.

In a yet another embodiment of the present invention, the blending process of the present invention comprises:
1) hydrogenating "forestry or wood" derived phytosterols to yield a product comprising from 60-85% beta-sitostanol and from 20-40% campestanol; and
2) blending this intermediary product with a non-hydrogenated plant (such as vegetable) derived phytosterol composition (such as that prepared and purified in accordance with the process of the present invention to yield a another desired composition having the ratios as defined herein for optimal therapeutic efficacy.

Hydrogenated wood derived phytosterols blended with non-hydrogenated vegetable or plant derived phytosterols are most preferably mixed in a ratio of 1:1.

In a yet another embodiment of the present invention, the blending process of the present invention comprises:
1) hydrogenating wood derived phytosterols to yield a product comprising from 60-85% beta-sitostanol and from 20-40% campestanol; and
2) blending this intermediary product with non-hydrogenated wood derived phytosterols (for example, as purified using the process described herein) to yield a another desired composition having the ratios as defined herein for optimal therapeutic efficacy.

Hydrogenated wood derived phytosterols blended with non-hydrogenated wood derived phytosterols are most preferably mixed in a ratio of 1:3.

In yet another preferred form, the blending process of the present invention comprises:
1) hydrogenating "plant" or "vegetable" derived phytosterols to yield a product comprising from 60-85% beta-sitostanol and from 20-40% campestanol; and
2) blending this intermediary product with a non-hydrogenated plant or vegetable derived phytosterol composition to yield a most desired composition having the ratios as defined herein for optimal therapeutic efficacy.

Hydrogenated plant derived phytosterols blended with non-hydrogenated plant derived phytosterols are most preferably mixed in a ratio of 1:1.

Furthermore, there are other "triplet" combinations or blends of wood derived and plant/vegetable derived phytosterols which are within the scope of the present invention. For example, wood derived and plant and plant derived phytosterols may be blended with either hydrogenated wood derived phytosterols or hydrogenated plant derived phytosterols to yield a composition with the desired ratio of plant sterol constituents. In one form of triplet blending, a wood derived phytosterol composition, a plant derived phytosterol composition and a hydrogenated plant derived composition (preferably vegetable or soy derived) is blended in a ratio of 1:1:2. In another form of triplet blending, a wood derived phytosterol composition, a plant derived phytosterol composition and a hydrogenated wood derived composition is blended in a ratio of 1:1:1.3.

Irrespective of whether it is the wood derived or the vegetable derived phytosterols which are hydrogenated, the blending step can be achieved by a number of different means. In a most preferred form, the constituent phytosterols/phytostanols are dissolved into a suitable solvent mixture and recrystallized to ensure uniformity in the end composition. Other "blending" methods are also appropriate.

Hydrogenation of the phytosterol composition in accordance with the present invention may be achieved by a number of different methods known and widely used in the art including the method described in Augustine and Reardon. The Palladium catalyzed hydrogenation of cholesterol. Org Prep. And Proceed. 1969;1:107-109. This reference is based on the use of Pd/C catalyst in organic solvents. Other suitable cataylsts include platinum, Raney nickel and palladium on alumina (latter used in Example 4, below).

The compositions of the present disclosure may be administered to animals, including humans, directly or may be incorporated into various vehicles for delivery such as pharmaceuticals, foods, beverages, nutraceuticals and the like, including dietary supplements and vitamin formulations for the treatment and/or prevention of dislipidemias, atherosclerosis and its consequences, strokes, heart attacks and peripheral vascular disease.

The phytosterol compositions described herein may be provided in the form of medications with suitable adjuvants or carriers. Furthermore, the compositions may be combined or prescribed with other known compounds which inhibit cholesterol synthesis, such as statins or other selected lipid-lowering agents, to decrease the necessary dosage, and hence toxicity, of these latter compounds.

While the following examples are intended to illustrate various aspects of the present invention and to assist in the process of purification, the blending techniques and the compositions so formed, they are not intended to limit the scope of invention as claimed herein.

### EXAMPLE 1 - Extraction from Soap (Reference)

One kilogram of soap was mixed with the extraction solvents in the following amounts: water (1L); acetone (2L); and hexane (5L) for 15 minutes under reflux conditions at 50 to 60 degrees C. After separation of the phases, the hexane layer (which is a mixture of hexane and acetone) was evaporated up to a final concentrate of 1kg (dry basis) dissolved in 1L of hexane. The evaporation was conducted under ambient pressure and temperature increasing to 95 degrees C. The hexane-acetone was recycled for further extractions. The water layer was evaporated to recover acetone. The resultant product is a concentrated extract (concentrated hexane layer).

### EXAMPLE 2 -Purification by Complexation in Petroleum Ether (Reference)

The hexane extract (150 g) obtained from Example 1 was dissolved in technical grade petroleum ether at 30-60 degrees C. Calcium Chloride dihydrate (20 g) was dissolved in 20 mL of water and added to the hexane extract. The mixture was heated up to 50 degrees C and the water was recovered under azeotropic evaporation. After three hours, 11 ml of water was recovered. The resultant yellow precipitate was filtered and washed with fresh petroleum ether. After drying, 58 g of the complex was recovered. The complex was then hydrolysed in hot distilled water at 80 degrees C for 30 minutes. The crude sterols were then filtered, washed with distilled hot water and dried. A mass of 31.8 g of final dry product was obtained having a phytosterol content of 77.83%. The overall yield for the purification was 76.6%.

### EXAMPLE 3 Purification of Tall Oil Soap (TOS) extract by Complexation in a mixture of Hexane-Acetone (Reference)

A tall oil soap extract (99.98 g) containing 32.6 % of sterols was dissolved in 98.56 g of mixture of hexane-acetone (75:25) (w/w). A solution of 10.44 g of anhydrous calcium chloride in 33.35 g of methanol was then added. The mixture was heated for 60 minutes at 40°C under stirring, after which an abundant precipitate is formed. The precipitate was then filtered and washed with 100 mL of same mixture of solvents (hexane-acetone). After drying at 65°C. 34.23 g of a white complex was recovered. The complex was then hydrolysed in hot distilled (200 mL) water at 80°C for one hour. The resultant sterols were filtered, washed with distilled water and finally dried. A mass of 25.413 g of final dry product (1.4% moisture content) was obtained having a minimum phytosterol content of 74.5 %. The overall phytosterol yield of the complexation-hydrolysis process for the extracted tall oil soap was 57.27 %.

### EXAMPLE 4 Purification of Tall Oil Soap (TOS) extract by Complexation in MIBK (methyl isobutyl ketone) (Reference)

A tall oil soap extract (100.01 g) containing 32.6 % of sterols was dissolved in 99.25 g of MIBK. A solution of 13.19 g of anhydrous calcium chloride in 39.09 g of methanol was then added. The mixture was heated for 60 minutes at 40°C under stirring, after what an abundant precipitate is formed. The precipitate was then filtered and washed with 100 mL of MIBK. After drying at 65°C, 36 g of the complex was recovered. The complex was then hydrolysed in hot distilled (200 mL) water at 80°C for one hour. The resultant sterols were filtered, washed with distilled water and finally dried. A mass of 33.26 g of final dry product (0.6% humidity) was obtained having a minimum phytosterol content of 60.9 %. The overall phytosterol yield of the complexation-hydrolysis process for the extracted tall oil soap was 61.14 %.

### EXAMPLE 5 Purification of Tall Oil Soap (TOS) by Complexation in MEK (methyl ethyl ketone) (Reference)

A tall oil soap extract (98.88 g) containing 32.6 % of sterols was dissolved in 105.11 g of MEK. A solution of 12.93 g of anhydrous calcium chloride in 38.95 g of methanol was then added. The mixture was heated for 60 minutes at 40°C under stirring, after what an abundant precipitate is formed. The precipitate was then filtered and washed with 100 mL of same mixture of solvents (MEK). After drying at 65°C, 40 g of a white complex was recovered. The complex was then hydrolysed in hot distilled (200 mL) water at 80°C for one hour. The resultant sterols were filtered, washed with distilled water and finally dried. A mass of 36.18 g of final dry product (5% humidity) was obtained having a minimum phytosterol content of 72.6 %. The overall phytosterol yield of the complexation-hydrolysis process for the extracted tall oil soap was 77.41 %.

### EXAMPLE 6 Purification of Vegetable Oil Distillate by Complexation In Methyl Ethyl Ketone (Reference)

A vegetable oil distillate (100.19 g) obtained from a deodorization process in soybean industry containing 7.59% of sterols was dissolved in 98.52 g of methyl ethyl ketone. A solution of 4.02 g of anhydrous calcium chloride in 11.92 g of methanol was then added. The mixture was heated for 60 minutes at 40°C under stirring, after what an abundant precipitate is formed. The precipitate was then filtered and washed with 100 mL of methyl ethyl ketone. After drying at 65°C, 17.38 g of the complex was recovered. The complex was then hydrolysed in hot distilled (200 mL) water at 80°C for one hour. The resultant sterols were filtered, washed with distilled water and finally dried. A mass of 9.79 g of final dry product was obtained having a minimum phytosterol content of 58.1%. The overall phytosterol yield of the complexation-hydrolysis process for the vegetal oil distillate was 73.17 %.

### EXAMPLE 7 Purification of a Pitch by Complexation in Methyl Ethyl Ketone (Reference)

A Pitch (100.18 g) coming from the fractional distillation of CTO (crude tall oil) manufacturing containing 6.96 % of sterols was dissolved in 98.19 g of methyl ethyl ketone. A solution of 10.42 g of anhydrous calcium chloride in 26.09 g of methanol was then added. The mixture was heated for 60 minutes at 40°C under stirring, after what a precipitate is formed. The precipitate was then filtered and washed two times with 100 mL of methyl ethyl ketone. After drying at 65°C, 11.297 g of the complex was recovered. The complex was then hydrolysed in hot distilled water (200 mL) at 80°C for one hour. The resultant sterols were filtered, washed with distilled water and finally dried. A mass of 8.271 g of final dry product was obtained having a minimum phytosterol content of 75.7%. The overall phytosterol yield of the complexation-hydrolysis process for the pitch was 89.79 %.

### EXAMPLE 8 Purification by Complexation in Mixture of Hexane and Acetone (Reference)

The hexane extract (142.9 g obtained from Example 1 was dissolved in 140 ml of a mixture of technical grade hexane and acetone in the volumetric ratio of 80/20. A solution of 20 g of calcium chloride dihydrate dissolved in 13 ml of water was then added. The mixture was evaporated for 130 minutes until 16.5 ml of the aqueous phase was recovered. The precipitate was then filtered and washed with a fresh mixture of hexane and acetone in the same volumetric ratio. After drying, 44.2 g of the complex was recovered. The complex was then hydrolysed in hot distilled water at 80 degrees C. The resultant sterols were filtered, washed with distilled water and finally dried. A mass of 30.4 grams of final dry product was obtained having a minimum phytosterol content of 69.8%. The overall phytosterol yield of the complexation-hydrolysis process was 70.7%.

### EXAMPLE 9 Hydrogenation Procedure (Reference)

Palladium on alumina (10% on alumina Aldrich; 100mg 2%w/w), isopropanol (Fisher Scientific, 50ml), distilled water (1% volume of the solvent) and the phytosterol mixture (5.0g) were added to a two-neck round bottom flask equipped with a magnetic stirring bar. The flask was submitted to a vacuum (water aspirator) and purged with hydrogen gas. This procedure was repeated three times.

The reaction mixture was then heated to 60-70 degrees C in an oil bath and then stirred for under a H2 atmosphere (1atm). The reaction was monitored by gas chromatography (GC) after 6 hours. Product ratio: campestanol and sitstanol 67.3%, starting material:28.6%, isomer4.1%.

Another portion of the catalyst (100mg, 2%w/w) was added to the reaction mixture and the hydrogenation was continued for 2 hours. GC analytical result: product 92%, starting material: 3.4%, isomer 4.6%.

The reaction mixture was filtered to recover the catalyst. The catalyst was then washed with isopropanol (50ml). Concentration of the combined solution by evaporation to recover the solvent afforded a white solid. The crude product thus obtained (5.6g, not dried) was recrystallized directly with EtOH (100ml) to provide an initial crop of crystals: first portion (4.1g); second portion (0.2g); overall yield: 86%.

Summary of Examples 3-8:
The molar ratio of CaCl₂ / sterols.
   In example 3, it is calculated to be 1.2.
   In example 4, it is calculated to be 1.5.
   In example 5, it is calculated to be 1.5.
   In example 6, it is calculated to be 1.98.
   In example 7, it is calculated to be 5.58.
The weight ration of Methanol / sterols.
   In example 3, it is calculated to be 1.02.
   In example 4, it is calculated to be 1.19.
   In example 5, it is calculated to be 1.21.
   In example 6, it is calculated to be 1.57.
   In example 7, it is calculated to be 3.74.
The performance for these conditions with our starting materials (i.e. TOS =3,4,5; Vegetable Oil distillate = 6 and PITCH = 7 is as follows:
   In example 3, Yield = 57.27% and purity = 74.5%
   In example 4, Yield = 61.14% and purity = 60.9%
   In example 5, Yield = 77.41 % and purity = 72.6%
   In example 6, Yield = 73.17% and purity = 58.1%
   In example 7, Yield = 89.79% and purity = 75.7%

### EXAMPLE 10 Blending of Phytosterols (Reference)

These steps of blending, concentrating and crystallizing the purified phytosterols may be used for "combining" any mixture in accordance with the present invention. The following were added to a 300L reactor

### Blending:

- hydrogenated phytosterol in isopropyl alcohol (IPA)-64.3kg @10% solids=6.4kg solids, 57.9kg IPA;
- phytosterols in IPA 103.1 kg @2% sotids=2.1 kg solids, 101kg IPA;
- dry wood derived non-hydrogenated phytosterol composition 7.1 kg solids;
- dry hydrogenated phytosterol composition 3.0kg solids;
- wet purified sterols, 20.4kg@85%solids=17.3kg solids, 3.1kg IPA.
- Total of 35.9kg solids (phytosterols) and 162kg IPA=197.9kg
   Weight ratio of 1 kg solids:4.5 kg IPA

This mixture was heated to 53 degrees (+/- 3degrees) C and held at that temperature for I hour to ensure all solids were dissolved.

### Concentrating

The sterol solution was concentrated in the reactor simply by pulling a vacuum on the reactor at 55 degrees C. The remaing weight in the reactor was 94kg, thus 68kg of IPA was evaporated from the solution. The resulting weight of the concentrated sterols was 1kg solid sterols:1.9 kg IPA.

### Crystallizing

The concentrated solution was pumped into a refrigerated tank (pumping temp was 55 degrees C). While slowly agitating, the sterol-IPA mixture was cooled from 55 degrees C down to 2.7 degrees C in 4 hours, 25 minutes. The sterol crystals were recovered by filtering through a plate and frame filter press fitted with 5 micron pore size filter papers. A total of 52.8 kg of wet crystals was recovered from the filter press. The crystals were transferred into an air deolventizer and desolventized for 48 hours at ambient temperature. The final IPA level of: the crystals was about 2.5%.

### EXAMPLE 11 Blending of vegetable derived phytosterol mixtures (Reference)

A vegetable derived phytosterol mixture comprising 44.36% beta-sitosterol, 1.08% sitostanol, 27.5% campesterol and 0.45% campestanol purified in accordance with the process of Example 3 was blended with a hydrogenated vegetable derived phytosterol mixture comprising 62.3% sitostanol and 32.2% campestanol in a ratio of 47.9 to 52.1 using the protocol outlined in Example 5. The resultant product was a novel "blended composition comprising 21.25% beta-sitosterol, 33.00% sitostanol, 13.17% campesterol and 16.99% campestanol.

### EXAMPLE 12 Blending of vegetable derived and wood derived phytosterol mixtures (Reference)

A vegetable derived phytosterol mixture comprising 44.36% beta-sitosterol, 1.08% sitostanol, 27.5% campesterol and 0.45% campestanol purified in accordance with the process of Example 3 was blended with a hydrogenated wood derived mixture comprising 73.47% sitostanol and 21.3% campestanol in a ratio of 55.9 to 44.1 using the protocol outlined in Example 5. The resultant product was a novel "blended composition comprising 24.80% beta-sitosterol, 33.00% sitostanol, 15.37% campesterol and 9.64% campestanol

### EXAMPLE 13 Blending of wood derived and vegetable derived phytosterol mixtures

A wood derived phytosterol mixture comprising 53.65% beta-sitosterol, 19.81% sitostanol, 15.76% campesterol and 0% campestanol purified in accordance with the process of Example 3 was blended with a hydrogenated vegetable derived mixture comprising 62.3% sitostanol and 32.2% campestanol in a ratio of 69 to 31 using the protocol outlined in Example 5. The resultant product was a novel "blended composition comprising 37.02% beta-sitosterol, 32.98% sitostanol, 10.87% campesterol and 9.98% campestanol.

### EXAMPLE 14 Blending of Wood derived phytosterol mixtures

A wood derived phytosterol mixture comprising 53.65% beta-sitosterol, 19.81% sitostanol, 15.76% campesterol and 0% campestanol, purified in accordance with the process of Example 3. was blended with a hydrogenated wood derived mixture comprising 73.47% sitostanol and 21.3% campestanol in a ratio of 75.45 to 24.55 using the protocol outlined in Example 5. The resultant product was a novel "blended" composition comprising 40.48% beta-sitosterol, 33.00% sitostanol, 11.89% campesterol and 5.23% campestanol.

## Claims

1. A process for preparing a phytosterol composition having from 30-80% beta-sitosterol, 4-25% campesterol, 10-60% sitostanol and 5-30% campestanol which comprises:
a) hydrogenating phytosterols from a first source to form an intermediary product comprising from 60-85% beta-sitostanol and from 20-40% campestanol; and
b) blending this intermediary product with phytosterols derived from a second source.

2. The process of claim 1 wherein the first source is either a plant-derived or forestry derived phytosterol composition.

3. The process of claim 1 wherein the second source is either a plant-derived or forestry derived phytosterol composition.

4. The process of claim 1 wherein the first source is vegetable or plant, the second source is forestry or wood and the ratio of the hydrogenated phytosterols to phytosterols from the second source is about 1:2.

5. The process of claim 1 wherein the first source is forestry or wood, the second source is vegetable or plant, and the ratio of the hydrogenated phytosterols to phytosterols from the second source is about 1:1.

6. The process of claim 1 wherein the first source is forestry or wood, the second source is forestry or wood and the ratio of the hydrogenated phytosterols to phytosterols from the second source is about 1:3.

7. The process of claim 1 wherein the first source is plant or vegetable, the second source is plant or vegetable and the ratio of hydrogenated phytosterols to phytosterols from the second source is about 1:1.

8. The process of claim 1 wherein the hydrogenation is carried out in the presence of a catalyst selected from Pd/C, platinum, Raney nickel and palladium on alumina.

9. The process of claim 1 wherein the first source is wood or plant and the phytosterols from the second source include both plant and wood derived phytosterols.

10. The process of claim 9 wherein the first source is plant and the ratio of wood derived phytosterol from the second source, plant derived phytosterol from the second source and hydrogenated phytosterols is about 1:1:2.

11. The process of claim 9 wherein the first source is wood, and the ratio of wood derived phytosterol from the second source, plant derived phytosterol from the second source and the hydrogenated phytosterols is about 1:1:1.3.

12. The process of claim 1 in which the blending is carried out by dissolving the hydrogenated phytosterols and the phytosterols from the second source into a solvent followed by recrystallisation.

## Patentansprüche

1. Verfahren zum Herstellen einer Phytosterinzusammensetzung mit 30-80% beta-Sitosterin, 4-25% Campesterin, 10-60% Sitostanol und 5-30% Campestanol, umfassend
a) Hydrierung von Phytosterinen aus einer ersten Quelle, um ein Zwischenprodukt zu bilden, das 60-85% beta-Sitostanol und 20-40% Campestanol umfasst; und
b) Mischen dieses Zwischenproduktes mit Phytosterinen, die aus einer zweiten Quelle herrühren.

2. Verfahren nach Anspruch 1, wobei die erste Quelle entweder eine aus Pflanzen oder der Forstwirtschaft stammende Phytosterinzusammensetzung ist.

3. Verfahren nach Anspruch 1, wobei die zweite Quelle entweder eine aus Pflanzen oder der Forstwirtschaft stammende Phytosterinzusammensetzung ist.

4. Verfahren nach Anspruch 1, wobei die erste Quelle Gemüse oder Pflanzen, die zweite Quelle Forstwirtschaft oder Holz und das Verhältnis der hydrierten Phytosterine zu Phytosterinen aus der zweiten Quelle ungefähr 1:2 ist.

5. Verfahren nach Anspruch 1, wobei die erste Quelle Forstwirtschaft oder Holz, die zweite Quelle Gemüse oder Pflanzen und das Verhältnis der hydrierten Phytosterine zu Phytosterinen aus der zweiten Quelle ungefähr 1:1 ist.

6. Verfahren nach Anspruch 1, wobei die erste Quelle Forstwirtschaft oder Holz, die zweite Quelle Forstwirtschaft oder Holz und das Verhältnis der hydrierten Phytosterine zu Phytosterinen aus der zweiten Quelle ungefähr 1:3 ist.

7. Verfahren nach Anspruch 1, wobei die erste Quelle Gemüse oder Pflanzen, die zweite Quelle Pflanzen oder Gemüse und das Verhältnis der hydrierten Phytosterine zu Phytosterinen aus der zweiten Quelle ungefähr 1: 1 ist.

8. Verfahren nach Anspruch 1, wobei die Hydrierung in Gegenwart eines Katalysators, ausgewählt aus Pd/C, Platin, Raney-Nickel und Palladium auf Aluminiumoxid ausgeführt wird.

9. Verfahren nach Anspruch 1, wobei die erste Quelle Holz oder Pflanzen ist und die Phytosterine aus der zweiten Quelle sowohl aus Pflanzen wie auch aus Holz herrührende Phytosterine umfassen.

10. Verfahren nach Anspruch 9, wobei die erste Quelle Pflanzen sind und das Verhältnis von aus Holz herrührendem Phytosterin aus der zweiten Quelle, aus Pflanzen herrührendem Phytosterin aus der zweiten Quelle und hydrierten Phytosterinen ungefähr 1:1:2 ist.

11. Verfahren nach Anspruch 9, wobei die erste Quelle Holz ist und das Verhältnis von aus Holz herrührendem Phytosterin aus der zweiten Quelle, aus Pflanzen herrührendem Phytosterin aus der zweiten Quelle und hydrierten Phytosterinen ungefähr 1:1:1,3 ist.

12. Verfahren nach Anspruch 1, bei dem das Mischen durch in Lösung Bringen der hydrierten Phytosterine und Phytosterine der zweiten Quelle in ein Lösungsmittel, gefolgt von Rekristallisation durchgeführt wird.

## Revendications

1. Procédé pour préparer une composition de phytostérols qui présente de 30 à 80 % de bêta-sitostérol, de 4 à 25 % de campestérol, de 10 à 60% de sitostanol et de 5 à 30% de campestanol, qui comprend les étapes qui consistent à :
a) hydrogéner des phytostérols provenant d'une première source pour former un produit intermédiaire qui comprend de 60 à 85 % de bêta-sitostanol et de 20 à 40% de campestanol, et
b) mélanger ce produit intermédiaire avec des phytostérols qui dérivent d'une deuxième source.

2. Procédé selon la revendication 1, dans lequel la première source est une composition de phytostérols qui dérivent de plantes ou une composition de phytostérols qui dérivent de la sylviculture.

3. Procédé selon la revendication 1, dans lequel la deuxième source est une composition de phytostérols qui dérivent de plantes ou une composition de phytostérols qui dérivent de la sylviculture.

4. Procédé selon la revendication 1, dans lequel la première source est une plante maraîchère ou une plante, la deuxième source est sylvicole ou est du bois, et le rapport entre les phytostérols hydrogénés et les phytostérols provenant de la deuxième source est d'environ 1 : 2.

5. Procédé selon la revendication 1, dans lequel la première source est sylvicole ou est du bois, la deuxième source est une plante maraîchère ou est une plante et le rapport entre les phytostérols hydrogénés et les phytostérols provenant de la deuxième source est d'environ 1 : 1.

6. Procédé selon la revendication 1, dans lequel première source est sylvicole ou est du bois, la deuxième source est sylvicole ou est du bois et le rapport entre les phytostérols hydrogénés et les phytostérols provenant de la deuxième source est d'environ 1 : 3.

7. Procédé selon la revendication 1, dans lequel la première source est une plante ou une plante maraîchère, la deuxième source est une plante ou une plante maraîchère et le rapport entre les phytostérols hydrogénés et les phytostérols provenant de la deuxième source est d'environ 1 : 1.

8. Procédé selon la revendication 1, dans lequel l'hydrogénation est réalisée en présence d'un catalyseur sélectionné parmi le Pd/C, le platine, le nickel de Raney et le palladium sur alumine.

9. Procédé selon la revendication 1, dans lequel la première source est du bois ou une plante et les phytostérols provenant de la deuxième source comprennent des phytostérols qui dérivent de plantes et des phytostérols qui dérivent de bois.

10. Procédé selon la revendication 9, dans lequel la première source est une plante et les proportions entre les phytostérols qui dérivent du bois de la deuxième source, les phytostérols qui dérivent de plantes de la deuxième source et les phytostérols hydrogénés sont d'environ 1 : 1 : 2.

11. Procédé selon la revendication 9, dans lequel la première source est du bois et les proportions entre les phytostérols qui dérivent du bois de la deuxième source, les phytostérols qui dérivent de la plante de la deuxième source et les phytostérols hydrogénés sont d'environ 1 : 1 : 3.

12. Procédé selon la revendication 1, dans lequel le mélange comprend l'étape qui consiste à dissoudre les phytostérols hydrogénés et les phytostérols de la deuxième source dans un solvant, suivie d'une étape qui consiste à recristalliser.
